# EUROPEAN PATENT SPECIFICATION

(11) **EP 1 251 815 B1**
(45) Date of publication and mention of the grant of the patent: **18.01.2017**
(21) Application number: 00984011.7
(22) Date of filing: 08.12.2000
(51) Int. Cl.: A61F 13/20, A61F 13/22

(54) **A PROCESS FOR PRODUCING DIGITAL TAMPON WITH UNEVEN NUMBER OF RIBS**
VERFAHREN ZUR HERSTELLUNG EINES TAMPONS MIT EINER UNGERADEN ANZAHL VON RIPPEN
PRPCÉDÉ POUR LA PRODUCTION D'UN TAMPON SANS APPLICATEUR COMPORTANT UN NOMBRE IMPAIR DE COTES

(30) Priority: 14.12.1999 EP 99124781
(43) Date of publication of application: 30.10.2002
(73) Proprietor: THE PROCTER & GAMBLE COMPANY, Cincinnati, OH 45202 (US)
(72) Inventor: FUCHS, Sybille, 60489 Frankfurt/M. (DE); PRICE, Samantha Jane, Teddington, Middlesex TW11 9AT (GB); HIRSCH, Uwe Thomas Michael Horst, 64347 Griesheim (DE); KREMER, Veronique Marie Josephine, 65824 Schwalbach (DE)
(74) Representative: Briatore, Andrea
(86) International application number: PCT/US2000/033207
(87) International publication number: WO 2001/043679

(56) References cited:
- WO-A-94/15564
- WO-A-97/23185
- DE-A1- 4 325 220
- US-A- 4 175 561
- US-A- 4 675 217
- US-A- 5 911 712

## Description

### Field of the invention

The present invention relates to compressed, radially-expanding fibrous digital tampons having an uneven number of ribs.

### Background of the invention

Generally tampons are manufactured from absorbent fibers, such as rayon, cotton, or a mixture of both fibers. In the case of digital tampons (i.e., tampons without any applicator aid) the volume of absorbent fibers necessary to provide sufficient absorption capacity must be highly compressed to form cylindrical tampons of sufficiently small size to allow comfortable insertion into the body. The compression is adequate to hold the tampon in cylindrical shape until insertion is completed. As a result the tampon when first inserted into the body is often compressed into a relatively non-conformable form with a relatively high initial density.

Most of the digital tampons currently available on the market are so called '8 flutes tampons'. These tampons have a circular cross-sectional core surrounded by 8 outwardly symmetrically extending ribs.

A drawback associated with such commercially available 8 flutes digital tampons is that they are not fully adapted to totally satisfy consumer needs in the sense that such tampons do not provide optimum protection for the heavy flow days and low flow days. Also such tampons might not be able to appropriately conform to the vaginal walls after insertion. Thus menstrual fluid may flow along the tampon's side and bypass its absorbent portions. Thus such tampons are susceptible to bypass leakage.

US5911712 relates to a digital tampon (20) for feminine hygiene, having a round dome (33) and a recovery tape (24), consists of a blank which is shaped by winding up a length of continuous random fibre web and which is pressed to produce a preform having a fibre core, longitudinal ribs and longitudinal grooves. The longitudinal ribs are pressed weakly to the final shape of the tampon (20; 28). The preform, before being pressed to the final shape of the tampon (20), is shaped as a result of the separate, but simultaneous radial pressing of directly adjacent sectors of its entire circumferential surface to form a longitudinal groove and a longitudinal rib respectively on each sector of the circumferential surface. The longitudinal groove and longitudinal rib assigned to each sector of the circumferential surface (182) are arranged respectively in succession in the same order in the circumferential direction of the preform. The fibre material is pressed radially to a lesser extent in the region of the end (22) provided with the recovery tape (24).

US4675217 relates to an absorbing product comprising a substantially cylindrical cartridge of a web material (13) with a first inner layer (25) and an intermediate layer (24) which merge into each other at a twisted portion (19) at one end of the cartridge, and a third outer layer (27) which connects to the first layer at another twisted portion (26) at the other end of the cartridge. The inner layer defines a closed cavity between the two twisted portions.

WO9723185 relates to a tampon formed of a substantially cylindrical mass of compressed fibers substantially enclosed in a fluid-permeable cover has improved ability to prevent early leakage. This tampon is capable of radially expanding upon exposure to a humid environment.

WO9415564 relates to a tampon (10) is provided, the surface of which is completely and securely covered with a porous over-wrap material (32). The tampon is formed by compressing the pledget (30), which comprises, prior to compression, a rolled length of absorbent material (31), having a conical depression (39) in one end. A permeable over-wrap material, in the form of a cylindrical envelope, completely surrounds the pledget.

US4175561 relates to Feminine Hygienic Pads with improved absorption properties for use in the interlabial space in the area of the female urethral meatus and vaginal meatus for absorbing urine and other undesirable exudations comprising an elongated fibrous pad base of cylindrical, elliptical, polygonal or similar regular shape, the surface of the pad base being formed with multiple flutings, a multiplicity of absorptive cells or pin-cushioning or being formed wholly or in part of shaggy material to increase the absorptive surface area of the pad.

DE- 4325220 discloses a process of compressing a tampon where the pressing edges have an eccentric orientation toward the central point of the press.

There is a need to provide digital tampons with optimum adaptation to a women needs during her menstrual cycle, i.e., low flow days (typically present on the beginning and end of the menstruation) and heavy flow days. Thus it is an object of the present invention to provide tampons particularly adapted to a particular portion of the menstrual cycle while maintaining optimum protection level. In other words there is a need of providing such digital tampons that would reduce or even prevent bypass leakage (early on and during use) while delivering effective absorption capacity.

The present invention achieves these objects by means of the features contained in the claims. It has now surprisingly been found that a digital tampon with 7 ribs which extend radially from the core of the tampon meets the needs of low flow days, whereas a digital tampon with 9 ribs is particularly adapted to meet the needs of heavy flow days, both while reducing bypass leakage due to a better adaptation to the morphology of the vagina.

The present invention selects 7 and 9 flutes digital tampons for an optimum level of protection of a women during her menstrual cycle and provides these tampons in a single package.

A drawback associated with the use of existing 8 flutes digital tampons on the market is that although the whole absorbency of the tampon might be appropriate, these tampons may fail in case of fast release of fluid also called gush occurrence, especially observed during heavy flow days. It has now surprisingly been found that the tampons of the present invention having an uneven number of 9 ribs allow faster acquisition of menstrual fluid. Indeed this execution provides a bigger circumferential surface area which results in a better utilisation of the absorbency of the fiber material per unit weight. Thus 9 flutes tampons are particularly suitable for the heavy flow days and gush occurrence.

Another drawback associated with the use of 8 flutes digital tampons on the market is that in case of low flow days, some women complain about dryness of the vagina resulting in uncomfortable feeling and of difficulty and unpleasantness of retrieval of the tampon. It has now surprisingly been found that the tampons herein having an uneven number of 7 ribs would slow down acquisition speed, thereby contributing to reduce the dryness felling in the vagina and facilitating the retrieval of the tampon after use.

The uneven number of ribs herein will participate in a non-circular cross-sectional expansion in use, i.e., upon exposure to wet conditions. Such a radial non-circular cross-sectional expansion is better adapted to the non-circular morphology of the vagina, and thereby reduces the occurrence of by pass leakage during use. Further any uneven number of ribs will help distribute radial forces in the tampon (during insertion and wearing) across a larger circumference than in an even rib design, due to their center point symmetry.

The 7 or 9 flutes tampons have been selected as they best combine the technical features of appropriate absorption capacity, appropriate fluid acquisition rate and reduction of bypass leakage with reasonable manufacturing constraints and cost. A higher number of flutes would result in more difficulties in the process in so far that very fine tool heads, so called press jaws, will be required to compress the tampon blank in its fluted shape. This would require a potential change of the material used for the press jaws and/or of the shape of the press jaws and/or cause a risk of cutting the tampon fibers. Also a lower number of ribs are believed to be less appealing to consumers.

### Summary of the invention

The present invention encompasses a process for producing a digital tampon according to the present invention in which a tampon blank is shaped by winding up a nonwoven material, preferably in presence of a fluid permeable covering material, the tampon blank is then pressed radially to form a tampon preform which has a central approximately cylindrical fibre core and 7 or 9 longitudinal ribs which extend radially outwards from the fiber core. The tampon blank is pressed radially relative to a longitudinal axis, which axis is an axis offset from the mid axis of the tampon blank, over 7 or 9 portions mutually adjacent in the circumferential surface of the tampon blank. This radial compression is such that the resulting preform is then allowed to expand into its final shape, preferably inside a cylindrical pipe, so that the outer surface of the tampon forms a soft and smooth cylindrical surface of at least slightly bigger diameter than the preform.

### Brief Description of the drawings

The invention is explained in more detail below by means of the diagrammatic drawings of an exemplary embodiments of the tampon, and of a process for their production with reference to apparatus illustrated schematically in the drawings, in which :
Figure 1 shows a perspective view of a tampon
Figure 2 shows an apparatus for producing the tampon according to Figure 1 in a longitudinal section
Figure 3 shows a cross-section through a pressed tampon preform along line V-V in Figure 2
Figure 4 shows a cross-section through a ready-pressed tampon along line VI-VI in Figure 2
Figure 5 shows a view of the exit side of a preforming press 36 for the preform, with closed asymmetric press jaws.

### Detailed description of the invention

The digital tampon of the present invention comprises a substantially cylindrical mass of compressed fibers, the tampon having a fibrous core substantially surrounding the central axis of the tampon and ribs which extend radially from the core, the number of ribs is an uneven number of 7 or 9.

The tampons of the present invention have the tendency to radially expand in a non-circular cross-sectional shape, upon exposure to a wet environment, typically upon contact with menstrual discharge in use, as a result of the uneven number of ribs, in contrast to even number of ribs. As used herein the term 'radially expand' relates to the expansion of the tampons herein in use conditions. These tampons expand primarily in a direction perpendicular to the central axis of the tampon. According to the present invention the tampons expand non-uniformly in at least two directions perpendicular to the central axis so as to be shaped in a non-circular cross section upon exposure in a wet environment.

Indeed the present invention is based on the finding that the tampons according to the present invention having an uneven number of ribs (7 or 9) have the tendency to radially expand in a non-circular cross-sectional shape when exposed to a wet environment (in contrast to tampons with an even number of flutes) thereby reducing bypass leakage. By 'bypass leakage' is meant the phenomenon in which menstruation fluid can escape between the body walls and tampon. Indeed the tampons herein have the ability in use to radially expand in a non-circular shape, i.e., a radial expansion which more adequately conform to the vagina anatomy and therefore helps reducing bypass leakage or even prevent it.

The present invention is further based on the finding that the 9 flutes digital tampon herein has a surface area of the circumference of the tampon which is bigger than those of a similar tampon (i.e., a tampon having same fiber weight, bulk density (weight of tampon per volume) and stability) having 8 ribs, this especially under use condition, i.e., upon exposure to a wet environment. This results in enhanced vaginal fluid capture at the circumference of the tampon and faster fluid absorption in use. The increased surface area at the circumference of the tampon is evidence of a better utilisation of the absorbency of the fiber material per unit weight. Thus such 9 flutes digital tampons for a given absorbency range are particularly adapted to heavy flow days and especially for gush occurrence, i.e., high amount of fluid discharge in short time sequence.

The tampons herein comprise a substantially cylindrical mass of compressed fibers. The tampons herein are made from nonwoven material/fibers which may be made of natural fibres such as cotton, wood pulp, jute and the like and/or processed fibres as regenerated cellulose, cellulose nitrate, cellulose acetate, rayon, polyester, polyvinyl alcohol, polyolefin, polyamine, polyamide, polyacrylonitrile, and the like. Other fibres in addition to the above fibres may be included to add desirable characteristics to the digital tampons. Preferably the tampon fibres are rayon or cotton and more preferably rayon. The fibres may have any useful cross-section. Preferred cross-sections include multi-limbed and non-limbed. Multi-limbed, regenerated cellulosic fibres have been commercially available for a number of years. These fibres are known to possess increased specific absorbency over non-limbed fibres. One commercial example of these fibres is the Galaxy® viscose rayon fibres available from Acordis England. These fibres are described in details in Courtaulds European application EP-A-1301874, the disclosure of which is herein incorporated by reference. These multi-limbed fibres are described as comprising a solid filament of regenerated cellulosic material having a decitex of less than 5.0 and multi-limbed cross-section, each limb having a length to width ratio of at least 2:1. The fibres are preferably staple length fibres having three or four limbs and a generally symmetrical cross-sectional shape, e.g., Y-, X, H, or T-shaped. A preferred cross-sectional shape is Y-shaped having an angle between limbs of about 120. Preferred regenerated cellulosic materials are viscose having a cellulose content of 5% to 12% and a caustic soda content of 4% to 10%. The fibres are preferably spun having a salt figure of 4.0 to 12.0.

Preferably the tampons herein include from 20% to 100% of multi-limbed fibres. Indeed these multi-limbed fibres are believed to accelerate the radial expansion properties of the tampons herein upon exposure to a wet environment and thus contribute to the benefit of the present invention by further reducing the occurrence of early bypass leakage. The fibres may be a mixture of multi-limbed fibres and non-limbed fibres. Preferably the tampons herein include from 20% to 100% of multi-limbed fibres and from 80% to 0% of the non-limbed fibers. More preferably the tampons herein include from 25% to 100% of multi-limbed fibres and from 75% to 0% of the non-limbed fibers. What ever the fibers used herein or mixtures of fibers used herein they are preferably blended to a substantially uniform mixture of fibers. These fiber blending operations are known to those skilled in the art. For example, the fibers can be continuously metered into a saw-tooth opener. The blended fibers can be transported, e.g., by air through a conduit a carding station to form a fibrous nonwoven material/web. This nonwoven material is then further processed as mentioned herein to form the tampon. In the tampon forming process the web/material is formed into a narrow, fibrous sliver and spirally wound to form the tampon blank. In addition a liquid permeable covering material is preferably wrapped around the tampon blank to substantially contain the fibrous absorbent portion of the tampon.

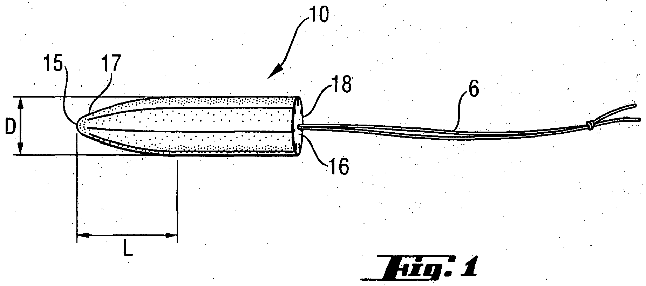

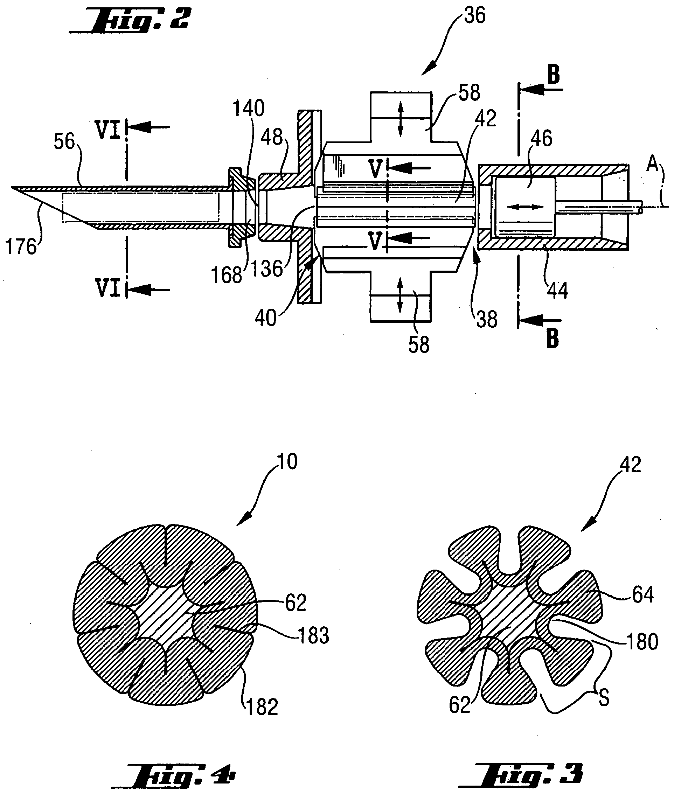

Figure 1 shows a tampon 10 for feminine hygiene formed from an approximately cylindrical blank which is shaped by winding up a portion of length of nonwoven material as described herein before, preferably in presence of a fluid permeable covering material. The circumferential surface of the tampon blank is then pressed radially relative to the longitudinal axis of the tampon blank, being either the mid-axis of the tampon blank or an axis which is offset from the mid axis of the tampon blank, over an uneven number of either 7 or 9 portions mutually adjacent in the circumferential direction of the tampon blank. The resulting tampon preform 42 (Figure 3) consists of a central approximately circular fiber core 62 of high compression and of longitudinal ribs 64 extending radially outwards from the fiber core. Thereafter the preform is formed into its final tampon form, typically by leaving it expand in a cylindrical expansion pipe, so as to form a soft cylindrical surface of at least a slightly bigger diameter than the preform.

In a preferred embodiment herein the tampon blank used as a starting point for the production of a tampon according to the present invention has a non-circular cross section, typically an ellipsoidal cross section. This can be achieved by winding up the nonwoven material, preferably in presence of the covering material directly in a tube that has the desired non-circular cross-sectional shape or by winding up these materials in a circular cross-sectional shaped tube and then incorporating the blank in another tube (see 44 in Figure 2) having the desired non-circular cross-sectional shape, preferably an ellipsoidal cross-section. In the preferred embodiment herein wherein the tampon is produced starting from a tampon blank having a non-circular cross section (preferably an ellipsoidal cross-section, i.e., that the different radius are non uniform as opposed to a circular cross section where all the radius are the same), this tampon, when wetted with fluid, endeavour to expand to the original, non-circular cross section of the tampon blank, and therefore provide further improved expansion properties. As a result the risk of bypass leakage in use is further reduced, as compared to a similar tampon having the same ribs configuration but which was produced by a tampon blank having a circular cross-section.

In the process of producing the tampons herein the circumferential surface of the tampon blank is preferably pressed radially relative to a longitudinal axis which is offset from the mid-axis of the tampon blank. Preferably no centralisation of the tampon blank in the press 36 is done before closing the press. In absence of any centralisation of the tampon blank in the press, the tampon blank while being fed in the press will have the tendency to fall into it so that when the press closes the tampon blank will not be pressed relative to its mid axis but relative to an axis which is offset from the mid axis of the tampon blank. As a result of the tampon blank being pressed radially relative to a longitudinal axis which is offset from the mid axis of the tampon blank, the adjacent portions formed (ribs), are non-uniform typically in the sense of having different widths. Appropriate regulation of the machinery equipment will allow to control/increase this phenomenon. The non-uniformity of the ribs will further contribute to a non-circular expansion during use and thus to more appropriate adaptation to the non-circular morphology of the vagina and hence further reduce by pass leakage.

Thereafter the resulting preform 42 is allowed to expand radially relative to the longitudinal axis of the preform thereby forming the final form of the tampon in such a way that the outer surface of the tampon forms a soft cylindrical surface of at least slightly bigger diameter than the diameter of the preform 42. This expansion process which results in a final tampon having a cylindrical surface of at least slightly bigger diameter than those of the preform further contributes to the expansion features of the uneven flutes tampon according to the present invention when exposed to a wet environment, thereby further reducing or preventing bypass-leakage during use conditions. The expansion step of the present process confers more conformability and a less dense outer end surface of the ribs of the tampon as opposed to a process wherein after compression of the preform the ribs are exposed to a low uniform pressure radial relative to the longitudinal axis of the preform to result in a tampon with a cylindrical surface of smaller diameter than the diameter of the preform. As a result of the present process the longitudinal ribs have the tendency to touch each other not only at the outer ends 182 but also along almost their entire facing side 183, preferably in way so that there is no holes between two longitudinal adjacent ribs 64. It is believed that as adjacent ribs touch each others along almost their entire facing side typically without any holes therebetween, an improved cross sectional fluid transport is observed between adjacent ribs. This contributes to a better use of the whole absorption capacity of the whole tampon and thus further participates to reduce fluid by pass leakage.

Figure 4 shows an enlarged cross-sectional representation of the fibre structure of the tampon according to the invention. It is possible to see clearly the central fibre core 62, from which extend outwards the longitudinal ribs 64 touching one another not only at their outer ends 182 but also along their facing side 183. The process according to the present invention guarantees a fibre core 62 having a high fibre compression despite the expansion phase after the compression phase. Indeed the digital tampons herein have a stability also called longitudinal crush resistance of at least 15N, preferably at least 20N and more preferably at least 30N. Typically the diameter of the tampon according to the invention is between 8 and 18mm in its final form.

Figure 2 illustrates an apparatus for producing a digital tampon 10 according to Figure 1. It comprises a preforming press 36 having an entry side 38 and an exit side 40 and serves for pressing the tampon blank to obtain a tampon preform 42. The preforming press 36 is equipped with press jaws 58 which are arranged in a plane perpendicular to the press axis A and are movable radially relative to the press axis A and which, in their closed position, each jaw is supported relative to two other one on their mutually opposite longitudinal sides. This counterbalancing of forces is in favour of overall stability. In the closed state of the press jaws 58, the end faces of the latter form an essentially cylindrical, stepped pressing surface, by means of which one of a plurality of directly adjacent sectors S of the entire circumferential surface of a tampon blank is loaded respectively for the purpose of pressing a longitudinal groove 180 and a directly adjacent longitudinal rib 64.

The preforming press 36 is preceded by a tube 44 having a pushing means 46. The tube is arranged so as to be movable to and for coaxially relative to the axis of the preforming press 36 and serves for shaping the tampon blank as desired, typically with a cross-sectional shape which is non-circular. The pushing means serves for pushing a respective tampon blank into the opened preforming press 36, the front of the tampon blank being at the front in the pushing-in direction corresponds to the insertion end of the resulting tampon and the rear of the tampon blank being at the end of the pushing-in direction corresponds to the withdrawal end of the resulting tampon.

The apparatus comprises, further, a guiding cone 48 which allows to guide the preform tampons from the press to the expansion pipe 56. This cone 48 is arranged coaxially relative to the press axis A of the preforming press 36 and is provided with an inlet orifice 136 and an outlet orifice 140. The diameter of the inlet orifice 136 of the cone is always bigger than the diameter of the outlet orifice 140 of the cone 48. The outlet orifice 140 of the cone 48 is dimensioned so as to not exercise any pressure on the tampon preform and accordingly is of the same cross sectional diameter or of a bigger cross-sectional diameter than the diameter of the tampon preform coming out of the preforming press 36. This guiding cone as for sole purpose the centralisation of the preform tampon coming out of the press, in the expansion pipe. No radial pressure is exercised on the tampon preform. Any internal shaping of this cone is suitable, e.g, spherical or even quadrangular.

The guiding cone 48 is followed by the conical expansion pipe 56 with an inlet orifice 168 and an outlet orifice 176. The diameter of the expansion pipe is uniform through its entire length and is bigger than the diameter of the tampon preform coming out of the preforming press. This allows expansion of the preform after the press 36 so that the outer ends of the longitudinal ribs form a soft circumferential surface of at least slightly bigger diameter.

Thereafter the tampon will be pressed co-axially to allow the formation of the tapered tip (machinery not shown in drawings). As a rule, there is an extra machinery equipment provided with a recess, known per se and therefore not shown, which resembles a spherical elongated cup and by means of which the insertion end of the tampon 10 is shaped in the manner of an elongated tip 15. In this case, there is provided in a likewise known way a counter-ram (not shown) which is moved up against the withdrawal end of the tampon 10 at the moment when a first ram is moved up against the insertion end of the tampon 10. Rams/counter-rams of this type are known in the art, and therefore there is no need to represent the driving elements for the rams. Typically the length of the head L is made of at least 50%, preferably more than 55%, more preferably from 60% to 150% and most preferably from 65% to 90% of the diameter D of the tampon (see Figure 1). This provides elongated tip shaped insertion end for optimal ease of insertion of the digital tampons of the present invention. D is the diameter of the tampon taken at its middle (i.e., at equal distance from the extremity of the withdrawal end and the extremity of the insertion end). L is the length of the head/elongated insertion end taken from its extremity up to the point where the diameter of the tampon starts to decrease as compared to the diameter D taken in the middle of the tampon.

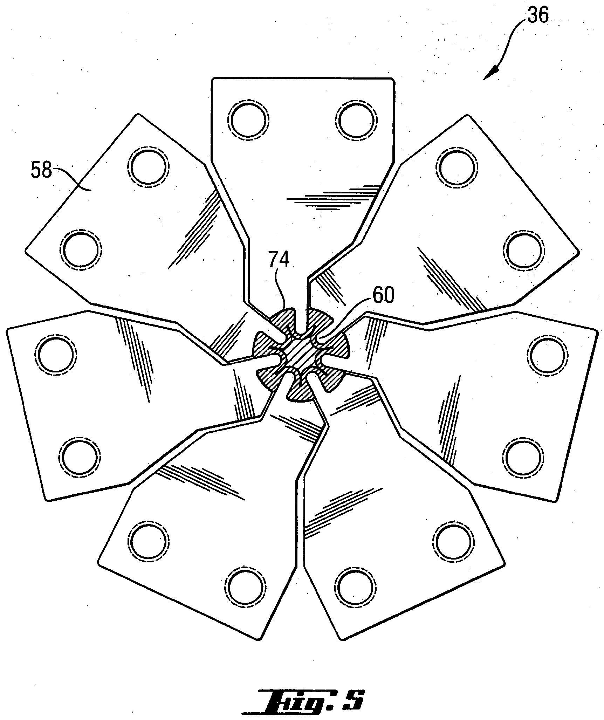

The preforming press 36 with seven or nine press jaws 58 is shown on a larger scale in its closed position in Figure 5 (exemplified is a press with seven press jaws). All the press jaws 58 are identical in respect of their shape and dimensions. Furthermore, the press jaws 58 are arranged so as to be movable synchronously into the closed or open position radially and concentrically relative to the press axis A (Figure 2). It is believed that the tampon blank in the press undergoes an overcompression, so that the circumferential length of the press orifice formed by the closed press jaws 58 is bigger than the circumference of the tampon blank taken at the outer ends of the ribs (see also the cross-section of the preform 42 Figure 3). The use of uneven number of press jaws 58 (seven or nine), in conjunction with pressing heads (not shown) of specific dimension, results in the best possible proportion by volume in a central fibre core 62 (Figure 3) and in the longitudinal ribs 64 of the preform 42 which extend radially from the fibre core 62. As a result of the simultaneous closing movement of the press jaws 58, a compaction, ensuring the high stability of the digital tampon 10 of a relatively small quantity of the fibre material is obtained coaxially relative to the longitudinal axis which axis is offset from the mid-axis of the tampon preform 42. As a result of the core overpressured process and preferably offsetting pressure longitudinal towards an axis which is typically not the mid axis of the preform a larger quantity of less compacted fibre material is available and can be activated immediately by fluid coming into contact with it in contrast to the conventional tampon produced by similar process as described typically in EP-A-611 562. Advantageously a greater proportion of the fibre material (ribs) can be activated by the fluid coming into contact with the tampon 10 and can be used for the purpose of higher fluid absorbency and expansion capacity.

The production of the tampon according to the invention by means of the apparatus in Figure 2 is carried out according to the following process: Once the tampon blank has been fed in the press narrow rib-shaped sectors of the circumferential surface of the tampon blank, which are parallel relative to the press axis A and which are preferably separated from one another by non equal circumferential angles as a result of the radial pressure of the press jaws 58 relative to an axis which is preferably offset from the mid axis of the tampon blank, are pressed radially relative to the press axis A as a result of the synchronous concentric closing movement of the press jaws 58, and the longitudinal grooves 180 are formed.

Simultaneously, by means of the pressing shoulders 74 of the same press jaws 58, which are located respectively on that side of the associated pressing head 60 directed in the anti-clockwise direction according to Figure 5, larger portions of the same sectors of the circumferential surface of the tampon blank are subjected to a pressing force to form the longitudinal ribs 64. Consequently, the preform 42 is obtained in one work cycle of the press jaws 58 moving jointly into the closed position. During the forming of the longitudinal grooves 180, the central fibre core 62 of approximately circular cross-section is produced, whilst the longitudinal ribs 64 extend radially outwards laterally of the longitudinal grooves 180.

After the preform 42 has been pressed, it is guided via the guiding cone 48 into the expansion pipe 56 where it expands in the final shape of the tampon with a diameter being at least slightly bigger than the diameter of the preform 42 coming out of the preforming press 36. Thereafter the tapered tip is formed. Two rams thereby exert an oppositely directed axial pressure on the tampon 10 which is consequently provided with the rounded elongated tip 15 for the insertion end 17 of the tampon 10 and with the finger recess 16 for the withdrawal end 18 of the tampon.

In a particular embodiment herein, the press jaws 58 and in particular the pressing shoulder 74 could be adapted so that they ensure that at least the fibre material located in the region of the withdrawal end of the tampon 10 has an overall lower compaction than the remaining fibre material of the tampon. Consequently, the retrieval string 6 of the tampon 10 can be embedded into a less compacted fibre material at the withdrawal end of the tampon 10 and can more easily be detached by hand from this fibre composite, with a finger recess thereby being formed or widened. In addition, a higher speed or expansion of the fibre material, which counteracts a leakage shortly after starting to use the tampon, is achieved in the region of the withdrawal end of the tampon.

In the above-described process for producing the tampon 10, it is also possible, if appropriate, to bring about the lower compression of the fibre material at the withdrawal end by means of the pressing surface, offset in a step-like manner, of the pressing heads 60. Moreover, it would be possible, if appropriate, to press the longitudinal grooves of the preform 42, starting at its front or insertion end, over its entire length to an increasingly lesser extent in the direction of the withdrawal end.

## Claims

1. A process for producing a digital tampon in which a tampon blank is shaped by winding up a nonwoven material, preferably in presence of a fluid permeable covering material, the tampon blank preferably has a non-circular cross section, more preferably an ellipsoidal cross section, the tampon blank is then pressed radially relative to a longitudinal axis of the tampon blank, said axis being an axis offset from the mid-axis of the tampon blank, to form a digital tampon having a central cylindrical fibre core and longitudinal ribs which extend radially outwards from the fiber core, the number of ribs being an uneven number of 7 or 9.

2. A process according to claim 1 wherein the tampon after having been radially pressed is left to expand typically in an expansion pipe, in its final shape so that the outer surface of the tampon forms a soft cylindrical surface of at least slightly bigger diameter.

## Patentansprüche

1. Verfahren zur Herstellung eines digitalen Tampons, bei dem ein Tamponrohling durch Aufwickeln eines Vliesmaterials, vorzugsweise unter Vorhandensein eines flüssigkeitsdurchlässigen Deckmaterials, geformt wird, vorzugsweise besitzt der Tamponrohling einen nicht kreisförmigen Querschnitt, mehr bevorzugt einen ellipsoidischen Querschnitt, anschließend wird der Tamponrohling radial relativ zu einer Längsachse des Tamponrohlings gepresst, wobei die Achse eine Achse ist, die von der Mittelachse des Tamponrohlings versetzt ist, um einen digitalen Tampon zu formen, der einen zentralen zylindrischen Faserkern und Längsrippen aufweist, die sich von dem Faserkern radial nach außen erstrecken, wobei die Anzahl der Rippen eine ungerade Zahl von 7 oder 9 ist.

2. Verfahren nach Anspruch 1, wobei der Tampon, nachdem er radial gepresst wurde, sich in seine endgültige Form ausdehnen gelassen wird, üblicherweise in einem Dehnungsrohr, so dass die Außenoberfläche des Tampons eine weiche zylindrische Oberfläche von wenigstens geringfügig größerem Durchmesser bildet.

## Revendications

1. Procédé de production d'un tampon digital dans lequel une ébauche de tampon est profilée par enroulement d'un matériau non tissé, de préférence en présence d'un matériau de couverture perméable aux fluides, l'ébauche de tampon a de préférence une coupe transversale non circulaire, plus préférablement une coupe transversale ellipsoïdale, l'ébauche de tampon est ensuite pressée en sens radial par rapport à un axe longitudinal de l'ébauche de tampon, ledit axe étant un axe décalé de l'axe médian de l'ébauche de tampon, pour former un tampon digital possédant une âme fibreuse cylindrique centrale et des nervures longitudinales qui s'étendent en sens radial vers l'extérieur à partir de l'âme fibreuse, le nombre de nervures étant un nombre impair valant 7 ou 9.

2. Procédé selon la revendication 1, dans lequel on laisse le tampon, après avoir été pressé en sens radial, s'étendre typiquement dans un tuyau d'expansion, sous sa forme finale de sorte que la surface externe du tampon forme une surface cylindrique souple d'un diamètre au moins légèrement plus grand.
